# EUROPEAN PATENT APPLICATION

(11) **EP 4 434 519 A1**
(43) Date of publication of application: **25.09.2024**
(21) Application number: 23305389.1
(22) Date of filing: 22.03.2023
(51) Int. Cl.: A61K 31/34, A61P 1/02, A61P 31/04, A61K 9/00, A61P 3/00, A61P 9/00, A61P 19/00, A61P 25/00, A61K 31/341

(54) **POLYOLS FOR THEIR USE IN THE PREVENTION AND TREATMENT OF DISEASES CAUSED BY ANAEROBIC PATHOGENIC MICROORGANISMS**

(71) Applicant: ROQUETTE FRERES, 62136 Lestrem (FR)
(72) Inventor: MENTINK, Léon, 59800 LILLE (FR); WILS, Daniel, 59190 MORBECQUE (FR)
(74) Representative: Plasseraud IP

(57) **Abstract**

The object of the present patent application pertains to the field of hexitols for their use in the prevention and/or treatment of diseases and/or symptoms caused by anaerobic pathogenic microorganisms, and more specifically to monoanhydrohexitols, dianhydrohexitols, or combinations thereof, for their use in the prevention and/or treatment of said diseases and/or symptoms. In one embodiment, the object is isosorbide for use in prevention and/or treatment of the diseases and/or symptoms associated with strictly anaerobic pathogenic bacteria, such as *Fusonucleatum bacterium, Porphyromonas gingivalis, Veillonella parvula* and *Parvimonas micra.*

## Description

### Technical Field

The present invention pertains to the field of hexitols for their use in the prevention and/or treatment of diseases and/or symptoms caused by anaerobic pathogenic microorganisms, and more specifically to monoanhydrohexitols, dianhydrohexitols, or combinations thereof, for use in the prevention and/or treatment of said diseases and/or symptoms.

### Background Art

In patent FR1761521, the applicant previously presented the non therapeutical use of a dianhydrohexitol such as isosorbide in oral hygiene, as being able to reduce the development of oral anaerobic bacterial strains which are non strictly anaerobic, such as *Streptococcus mutans, Lactobacillus gasseri, Actinomyces naeslundii,* and *Actinomyces oris.* Isosorbide was thus proposed for the replacement of antiseptics in oral care hygiene.

### Technical Problem

Recently, a link between the presence of certain anaerobic pathogenic microorganisms in the oral cavity of human beings and some diseases and/or their symptoms was established. More specifically, the anaerobic pathogenic microorganisms are strictly anaerobic ones, such as *Fusobaceterium nucleatum, Porphyromonas gingivalis.* They can develop in locations inside the oral cavity where no oxygen is available, such as between the tooth and the dental plaque, also called tartar, formed upon said tooth, or between the gum and the tooth. From there, they may cause two types of problems, mainly thanks to their protease activity. First one is oral infections, leading to periodontal diseases such as periodontitis. Second one is an ectopic colonization of other parts or organs in the body because they can enter the bloodstream from the infected locations in the oral cavity. They thus can spread to every organ in the body, such as the brain, the heart, and the intestines, where they may cause diseases, such as cancers, brain inflammation, Alzheimer's disease, preterm birth, autoimmune diseases, cardiovascular diseases, diabetes, obesity, insulin resistance, rheumatoid arthritis, inflammatory bowel diseases, or respiratory tract inflammation.

A way to prevent and/or treat the diseases and/or symptoms associated with strictly anaerobic pathogenic microorganisms may be to prevent and/or treat the infection of the oral cavity by said strictly anaerobic pathogenic microorganisms. This is an object of the present patent application.

### Brief Description of Drawings

Other features, details and advantages will be shown in the following detailed description and on the figures, on which:
**Fig. 1**

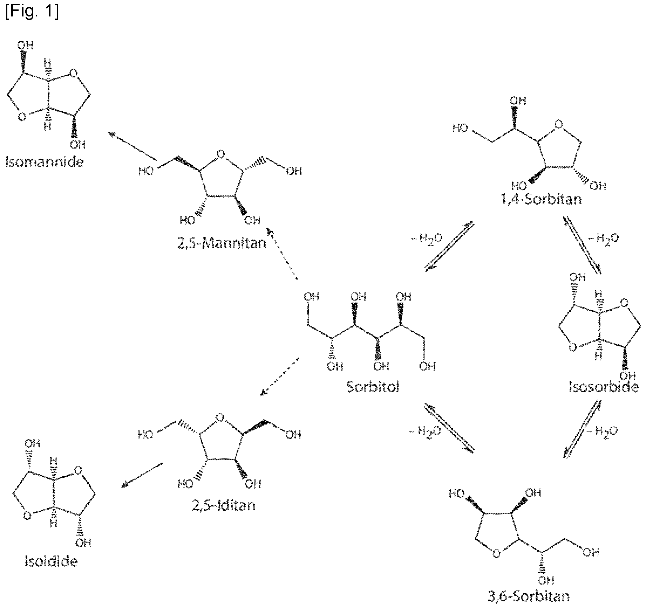

   [Fig. 1] shows chemical structures of sorbitol, the monoanhydrohexitols and dianhydrohexitols obtained from it;
**Fig. 2**

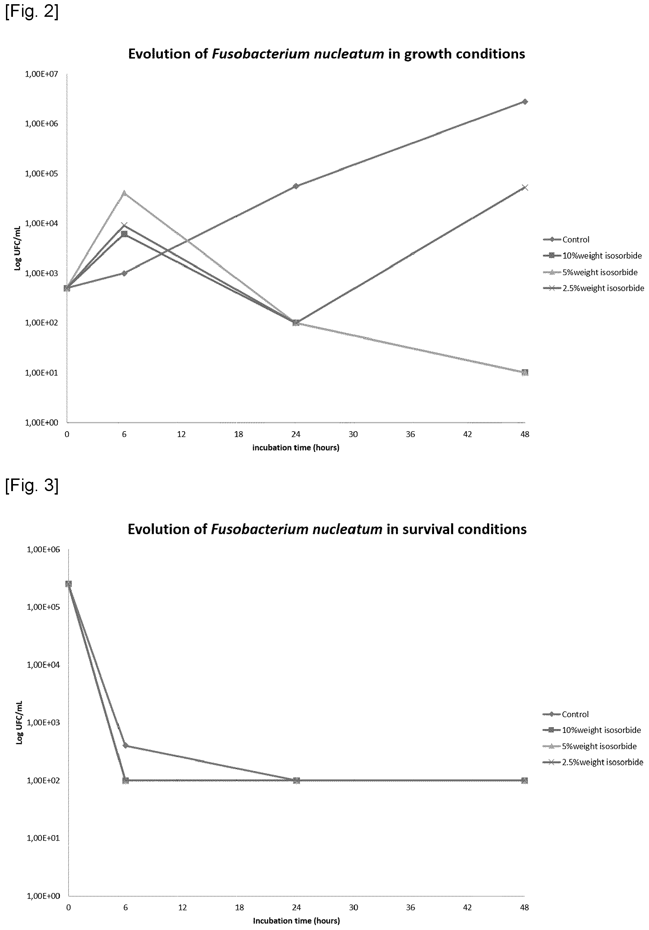

   [Fig. 2] shows the evolution of populations of *Fusobacterium nucleatum* in growth conditions for isosorbide content of 2.5, 5 and 10 % weight;
**Fig. 3**
   [Fig. 3] shows the evolution of populations of *Fusobacterium nucleatum* in survival conditions for isosorbide content of 2.5, 5 and 10 % weight;
**Fig. 4**

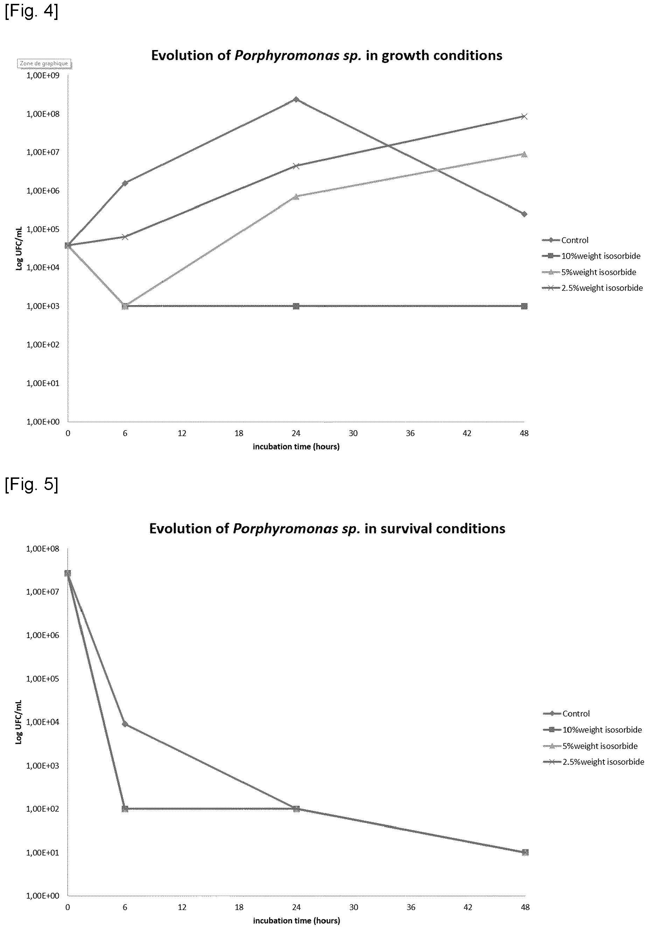

   [Fig. 4] shows the evolution of populations of *Porphyromonas sp.* in growth conditions for isosorbide content of 2.5, 5 and 10 % weight;
**Fig. 5**
   [Fig. 5] shows the evolution of populations of *Porphyromonas sp.* in survival conditions for isosorbide content of 2.5, 5 and 10 % weight;
**Fig. 6**

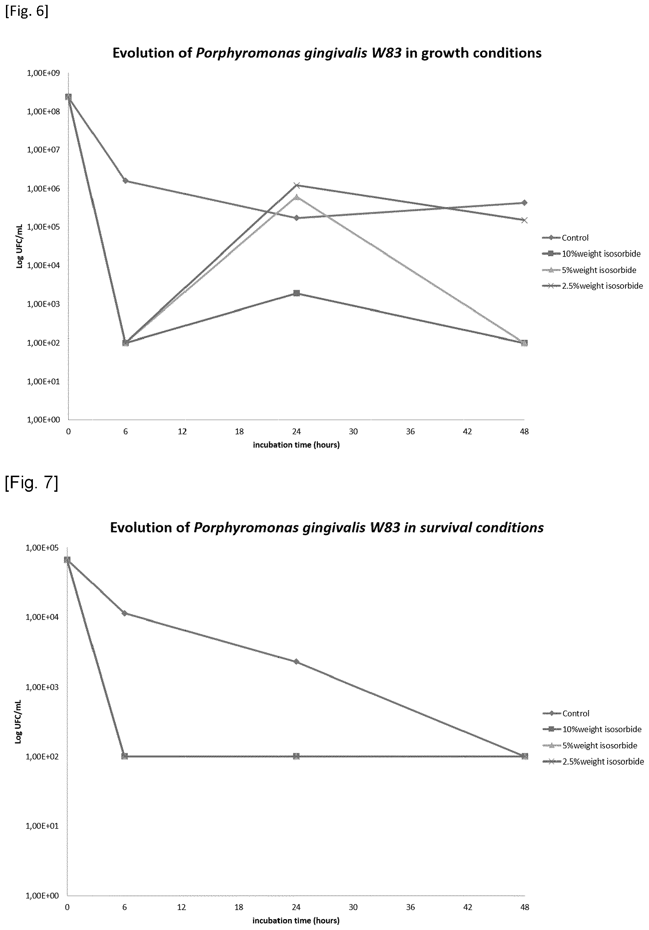

   [Fig. 6] shows the evolution of populations of *Porphyromonas gingivalis W83* in growth conditions for isosorbide content of 2.5, 5 and 10 % weight;
**Fig. 7**
   [Fig. 7] shows the evolution of populations of *Porphyromonas gingivalis W83* in survival conditions for isosorbide content of 2.5, 5 and 10 % weight;
**Fig. 8**

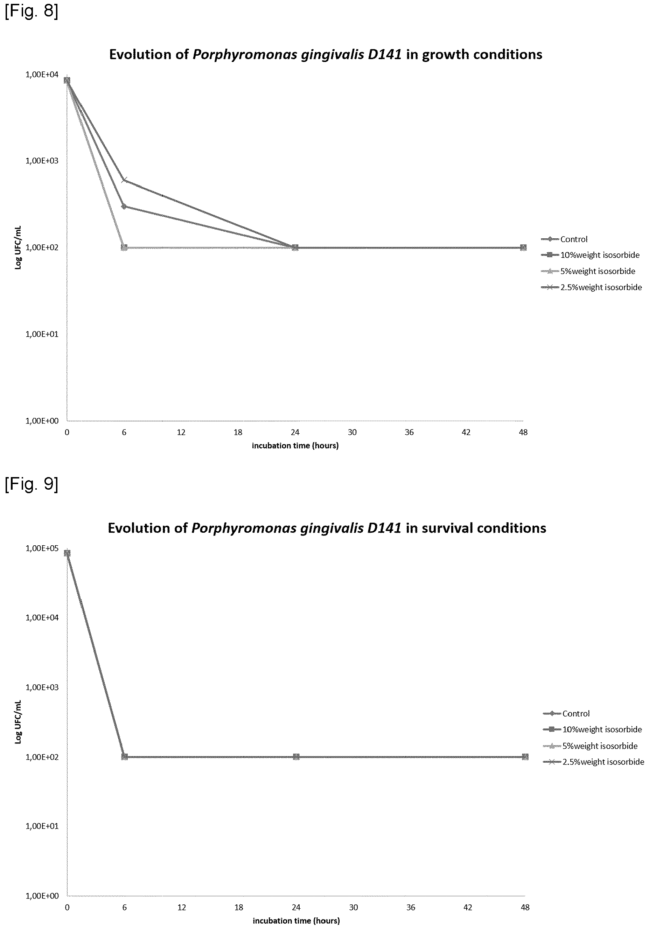

   [Fig. 8] shows the evolution of populations of *Porphyromonas gingivalis D141* in growth conditions for isosorbide content of 2.5, 5 and 10 % weight;
**Fig. 9**
   [Fig. 9] shows the evolution of populations of *Porphyromonas gingivalis D141* in survival conditions for isosorbide content of 2.5, 5 and 10 % weight;
**Fig. 10**

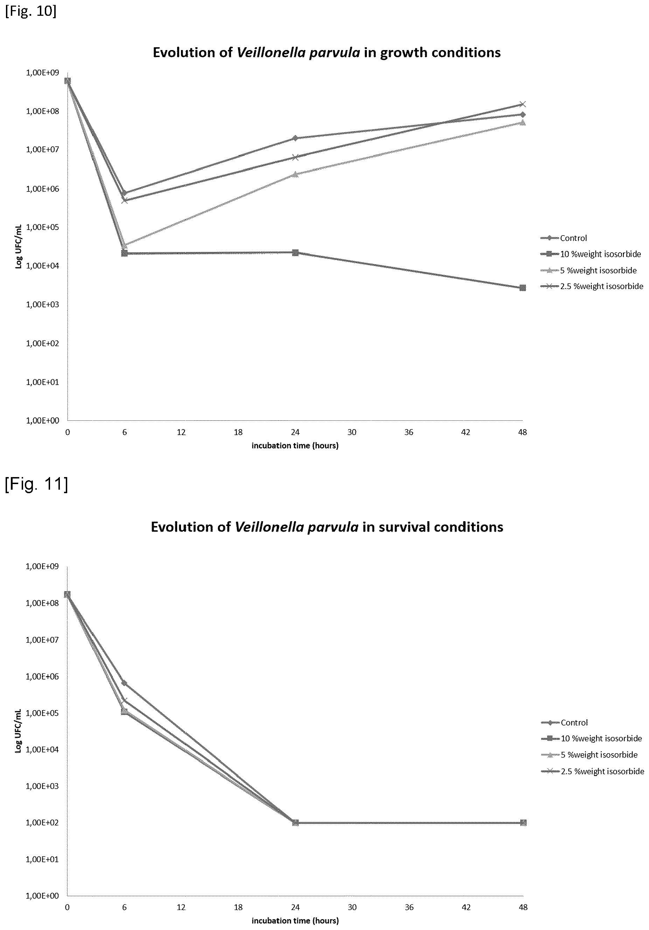

   [Fig. 10] shows the evolution of populations of *Veillonella parvula* in growth conditions for isosorbide content of 2.5, 5 and 10 % weight;
**Fig. 11**
   [Fig. 11] shows the evolution of populations of *Veillonella parvula* in survival conditions for isosorbide content of 2.5, 5 and 10 % weight;
**Fig. 12**

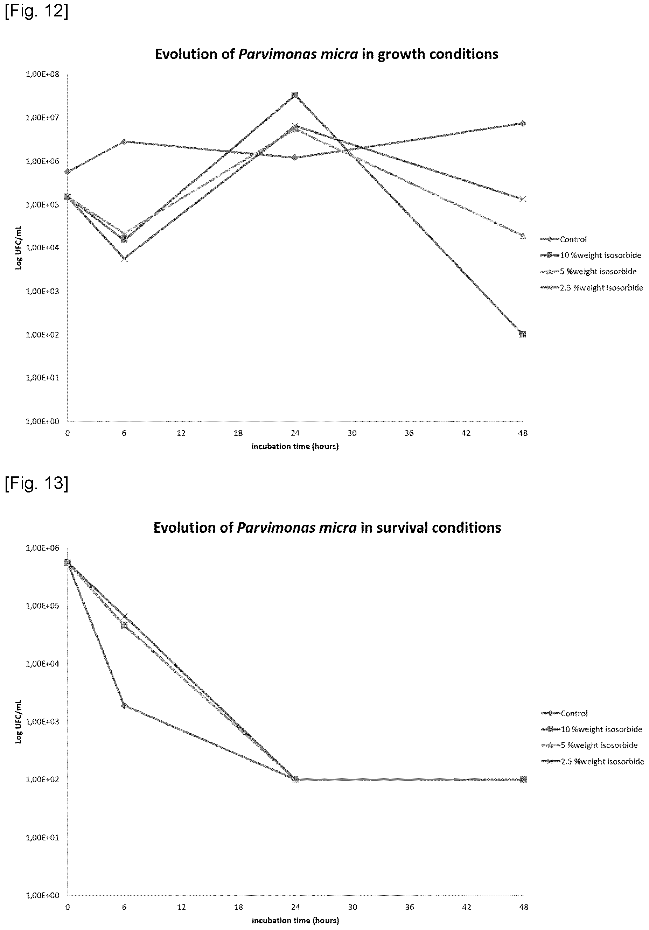

   [Fig. 12] shows the evolution of populations of *Parvimonas micra* in growth conditions for isosorbide content of 2.5, 5 and 10 % weight;
**Fig. 13**
   [Fig. 13] shows the evolution of populations of *Parvimonas micra* in survival conditions for isosorbide content of 2.5, 5 and 10 % weight;

### Summary of the invention

A first object of the present application is a polyol for use in prevention and/or treatment of diseases and/or symptoms associated with pathogenic microorganisms in a subject in need thereof.

In one aspect, the pathogenic microorganisms are anaerobic pathogenic microorganisms. In another aspect, the pathogenic microorganisms are strictly anaerobic pathogenic microorganisms.

In another aspect, the pathogenic microorganisms are bacteria of the genus *Fusobacterium,* such as bacteria of the species *Fusobacterium nucleatum, Fusobacterium necrophorum,* or *Fusobacterium polymorphum.*

In another aspect, the pathogenic microorganisms are bacteria of the genus Porphyromonas, such as bacteria of the species Porphyromonas gingivalis.

In another aspect, pathogenic microorganisms are bacteria of the genus *Veillonella,* such as bacteria of the species *Veillonella parvula, Veillonella dispar,* or *Veillonella atypica.*

In another aspect, pathogenic microorganisms are bacteria of the genus *Parvimonas,* such as bacteria of the species *Parvimonas micra.*

In another aspect, the polyol is an hexitol. In another aspect, the polyol is a monoanhydrohexitol, such as 1,4-Sorbitan, 2,5-Mannitan, 2,5-Iditan, 3,6-Sorbitan, or combinations thereof. In another aspect, the polyol is a dianhydrohexitol, such 1,4-3,6-dianhydrohexitols. In particular, said 1,4-3,6-dianhydrohexitols is isosorbide, isomannide, isoidide, or combinations thereof. In another aspect, the polyol is isosorbide.

In yet another aspect, the diseases and/or symptoms are cancer, such as a tongue cancer, an esophagus cancer, a stomach cancer, an intestine cancer, a colon cancer, or a systemic cancer ; brain inflammation, Alzheimer's disease, preterm birth, autoimmune diseases, cardiovascular diseases, such as atherosclerosis and/or coronary disease ; diabetes, obesity, insulin resistance, rheumatoid arthritis, inflammatory bowel diseases, or respiratory tract inflammation.

In particular, the diseases and/or symptoms are inside the oral cavity, such as oral lichen planus, oral cancer, or periodontal diseases such as periodontitis.

In particular, the symptoms are inflamed gums, bleeding gums, pain in the gum, teeth and/or jaw at rest, pain in the gum, teeth and/or jaw during chewing, loose teeth, loss of teeth, fever, halitosis

A second object of the present application is a composition comprising a polyol for use in prevention and/or treatment of diseases and/or symptoms associated with pathogenic microorganisms in a subject in need thereof, as described in the first object of the present application.

In another aspect, the composition is a liquid, a gel, a compact or loose powder, a solid, or a film.

In another aspect, the composition is a toothpaste, a toothgel, a capsule, a soft-gel capsule, a tablet such as oral care tablet, an oral care film, a mouthwash, an oral spray, or a sweet such as a candy.

In another aspect, the content of said polyol in said composition is at least 0,5 % weight, at least 1 % weight, at least 5 % weight, or at least 10 % weight of the total weight of said composition.

In another aspect, the composition is rinsed off, or is left on, after its administration.

In another aspect, the composition is applied into, or onto, a dental mould, an oral care patch, a dental floss, or a dental strip.

In another aspect, the composition comprises less than 1 % weight of dimethylisosorbide of the total weight of said composition or is free of dimethylisosorbide.

### Detailed description

### Polyol

Polyols are alcohols comprising at least two alcoholic functions, i.e. at least two hydroxyl -OH groups. In an aspect, the polyol comprises at least two atoms of carbon, at least three atoms of carbon, at least four atoms of carbon, at least five atoms of carbon, or at least six atoms of carbon.

### Hexitol

An hexitol is a polyol comprising six atoms of carbon and six alcohol functions, such as sorbitol, mannitol and iditol.

### Anhydrohexitol

An anhydrohexitol is an hexitol comprising at least one heterocycle with an oxygen atom, usually obtained from hexitol by dehydration under acidic conditions together with a catalyst. A monoanhydrohexitol is a heterocyclic hexitol comprising one cycle, such as 1,4-Sorbitan, 2,5-Mannitan, 2,5-Iditan, 3,6-Sorbitan. A dianhydrohexitol is a heterocyclic hexitol comprising two cycles, such as 1,4-3,6-dianhydrohexitol, which can be isosorbide, isomannide, isoidide.

### Pathogenic microorganism

A pathogenic microorganism is a bacteria, a fungus or a protozoan that cause one or more disease in mammals, such as humans or animals, when said pathogenic microorganisms infect said mammals.

In one embodiment, the pathogenic microorganism is anaerobic.

An anaerobic microorganism may be tolerant the presence of oxygen in its surroundings, and is then called non strictly anaerobic, or may be completely intolerant to the presence of oxygen in its surroundings, and is then called strictly anaerobic. "Tolerant" means that non strictly anaerobic microorganisms may grow in mediums, such as parts of the human body like the oral cavity, which contains few oxygen. "Intolerant" means that strictly anaerobic microorganisms may not grow at all in said low-oxygen mediums, and therefore can survive only a limited period of time in said low-oxygen mediums. The strictly anaerobic microorganisms need a medium which is free of oxygen to be able to survive long enough and to grow.

In another embodiment, the pathogenic microorganism is strictly anaerobic.

### Subject in need thereof

The term "subject", as used herein, means a mammal, including for example livestock (for example cattle, horses, pigs, and sheep) and humans. In one embodiment the subject is a human. In one embodiment the subject is female. In one embodiment the subject is male. In another embodiment, the subject is a dog (such as a member of the genus Canis) or a cat (such as a member of the genera Feiis or Panthera).

### Composition comprising a polyol

The composition according to the second object of the present application may comprise at least one polyol according to the first object of the present application. Such a composition may be, or may be used as an ingredient for the preparation of, a food product, a food ingredient, a dietary supplement, a cosmetic product, a medical food, a vaccine or a pharmaceutical acceptable composition or formulation.

### Pharmaceutical composition

The composition according to second object of the present application may be used as - or in the preparation of - a pharmaceutical composition or formulation. Here, the term "pharmaceutical" is used in a broadsense - and covers pharmaceuticals for humans as well as pharmaceuticals for animals (i.e. veterinary applications).

In an embodiment, the pharmaceutical acceptable composition is a medicament.

The pharmaceutical composition can be for therapeutic purposes - which may be curative or palliative or preventative in nature. The pharmaceutical composition may even be for diagnostic purposes.

In an embodiment of the present invention, the medicament is for oral administration.

A pharmaceutically acceptable composition or support may be for example a formulation or support in the form of creams, foams, gels, lotions, and ointments of compressed tablets, tablets, capsules, ointments, suppositories or drinkable solutions.

### Food Product

In one embodiment, the composition according to the second object of the present application is used in a food product, such as a food supplement, a drink or a powder based on milk. Here, the term "food" is used in a broad sense and covers food for humans as well as food for animals (i.e. a feed). In an aspect, the food is for human consumption.

The food may be in the form of a solution or as a solid, depending on the use and/or the mode of application and/or the mode of administration.

### Dietary Supplement

The composition according to the second object of the present application may take the form of dietary supplements or may themselves be used in combination with dietary supplements, also referred to herein as food supplements. The term "dietary supplement" as used herein refers to a product intended for ingestion that contains a "dietary ingredient" intended to add nutritional value or health benefits to (supplement) the diet. A "dietary ingredient" may include (but is not limited to) one, or any combination, of the following substances: bacteria, a probiotic (e.g. probiotic bacteria), a vitamin, a mineral, a herb or other botanical, an amino acid, a dietary substance for use by people to supplement the diet by increasing the total dietary intake, a concentrate, metabolite, constituent, or extract.

Dietary supplements may be found in many forms such as tablets, capsules, soft gels, gel caps, liquids, or powders. Some dietary supplements can help ensure an adequate dietary intake of essential nutrients; others may help prevent or treat diseases.

### Medical food

The composition according to the second object of the present application may take the form of medical foods. By "medical food" it is meant a food which is formulated to be consumed or administered with or without the supervision of a physician and which is intended for a specific dietary management or condition for which distinctive nutritional requirements, based on recognized scientific principles, are established by medical evaluation.

### Cosmetic product

The composition according to the second object of the present application may take the form of cosmetic products, such as a skin care product, a hair care product, a make-up product, or a hygiene product.

In one embodiment, the cosmetic product is a hygiene product. In particular, the hygiene product may be an oral care tablet, an oral care film, a mouthwash, an oral spray, a dental mould, an oral care patch, a dental floss, or a dental strip.

### Specific numbered embodiments of the invention

Embodiment 1. Polyol for use in prevention and/or treatment of diseases and/or symptoms associated with pathogenic microorganisms in a subject in need thereof.

Embodiment 2. The polyol for use according to embodiment 1, wherein the pathogenic microorganisms are anaerobic pathogenic microorganisms.

Embodiment 3. The polyol for use according to embodiment 2, wherein the anaerobic pathogenic microorganisms are strictly anaerobic pathogenic microorganisms.

Embodiment 4. The polyol for use according to any one of embodiments 1-3, wherein the polyol is administered in the oral cavity.

Embodiment 5. The polyol for use according to any one of embodiments 1-3, wherein the polyol is administered on the skin, eyes, ears, or genital mucous membranes, of said subject.

Embodiment 6. The polyol for use according to any one of embodiments 1-5, wherein said pathogenic microorganisms are bacteria of the genus Fusobacterium.

Embodiment 7. The polyol for use according to preceding embodiment 6, wherein the bacteria of the genus Fusobacterium are of the species Fusobacterium nucleatum.

Embodiment 8. The polyol for use according to preceding embodiment 6, wherein the bacteria of the genus Fusobacterium are of the species Fusobacterium necrophorum.

Embodiment 9. The polyol for use according to preceding embodiment 6, wherein the bacteria of the genus Fusobacterium are of the species Fusobacterium polymorphum.

Embodiment 10. The polyol for use according to any one of embodiments 1-5, wherein said pathogenic microorganisms are bacteria of the genus Porphyromonas.

Embodiment 11. The polyol for use according to preceding embodiment 10, wherein the bacteria of the genus Porphyromonas are of the species Porphyromonas gingivalis.

Embodiment 12. The polyol for use according to any one of embodiments 1-5, wherein said pathogenic microorganisms are bacteria of the genus Veillonella.

Embodiment 13. The polyol for use according to preceding embodiment 12, wherein the bacteria of the genus Veillonella are of the species Veillonella parvula.

Embodiment 14. The polyol for use according to preceding embodiment 12, wherein the bacteria of the genus Veillonella are of the species Veillonella dispar.

Embodiment 15. The polyol for use according to preceding embodiment 12, wherein the bacteria of the genus Veillonella are of the species Veillonella atypica.

Embodiment 16. The polyol for use according to any one of embodiments 1-5, wherein said pathogenic microorganisms are bacteria of the genus Parvimonas.

Embodiment 17. The polyol for use according to preceding embodiment 16, wherein the bacteria of the genus Parvimonas are of the species Parvimonas micra.

Embodiment 18. The polyol for use according to any of the preceding embodiments, wherein the polyol is an hexitol.

Embodiment 19. The polyol for use according to any of the preceding embodiments, wherein the polyol is a monoanhydrohexitol.

Embodiment 20. The polyol for use according to any of the preceding embodiments, wherein the polyol is selected from 1,4-Sorbitan, 2,5-Mannitan, 2,5-Iditan, 3,6-Sorbitan, or combinations thereof.

Embodiment 21. The polyol for use according to any one of embodiments 1-18, wherein the polyol is a dianhydrohexitol.

Embodiment 22. The polyol for use according to any one of embodiments 1-18 and 21, wherein the polyol is a 1,4-3,6-dianhydrohexitol.

Embodiment 23. The polyol for use according to any one of embodiments 1-18 and 21-22, wherein the polyol is selected from isosorbide, isomannide, or isoidide.

Embodiment 24. The polyol for use according to any one of the embodiments 1-18 and 21-23, wherein the polyol is isosorbide.

Embodiment 25. The polyol for use according to any one of the preceding embodiments, wherein said diseases and/or symptoms are cancer, brain inflammation, Alzheimer's disease, preterm birth, autoimmune diseases, cardiovascular diseases, diabetes, obesity, insulin resistance, rheumatoid arthritis, inflammatory bowel diseases, or respiratory tract inflammation.

Embodiment 26. The polyol for use according to embodiment 25, where said cancer is a tongue cancer, an esophagus cancer, a stomach cancer, an intestine cancer, a colon cancer, or a systemic cancer.

Embodiment 27. The polyol for use according to embodiment 25, where said cardiovascular diseases are atherosclerosis and/or coronary disease.

Embodiment 28. The polyol for use according to any one of embodiments 1-24, wherein said diseases and/or symptoms are inside the oral cavity.

Embodiment 29. The polyol for use according to embodiment 28, wherein said diseases and/or symptoms are oral lichen planus, oral cancer, or periodontal diseases.

Embodiment 30. The polyol for use according to embodiment 29, wherein said periodontal disease is periodontitis.

Embodiment 31. The polyol for use according to embodiment 28, wherein said symptoms are inflamed gums, bleeding gums, pain in the gum, teeth and/or jaw at rest, pain in the gum, teeth and/or jaw during chewing, loose teeth, loss of teeth, fever, halitosis.

Embodiment 32. The polyol for use according to any one of preceding embodiments, wherein said polyol slows down or inhibits the growth of said pathogenic microorganisms.

Embodiment 33. The polyol for use according to any one of preceding embodiments, wherein said polyol slows down the formation and/or reduces a biofilm formed by said pathogenic microorganisms.

Embodiment 34. The polyol for use according to embodiment 33, wherein said biofilm is formed in the oral cavity.

Embodiment 35. The polyol for use according to any one of preceding embodiments, wherein said polyol is applied during pathogens's growth conditions and/or during pathogens's survival conditions.

Embodiment 36. The polyol for use according to embodiment 35, wherein the pathogen's growth conditions correspond to the period following ingestion of food, such as after a meal, and the pathogen's survival conditions correspond to the condition where no nutrients are available to the subject.

Embodiment 37. The polyol for use according to any one of the preceding embodiments, wherein said polyol is administered at least once a day, at least twice a day, at least three times a day, at least four times a day.

Embodiment 38. The polyol for use according to any one of the preceding embodiments, wherein said polyol is administered for at least 30 seconds, at least 1 minute, at least 2 minutes, at least 3 minutes, at least 5 minutes, at least 10 minutes, at least 30 minutes, at least 1 hour, at least 4 hours, at least 6 hours, at least 8 hours, at least 12 hours, at least 16 hours, at least 20 hours, at least 24 hours.

Embodiment 39. Composition comprising a polyol for use in prevention and/or treatment of diseases and/or symptoms associated with pathogenic microorganisms in a subject in need thereof, as described in embodiments 1-38.

Embodiment 40. The composition according to embodiment 39, wherein said composition is a liquid, a gel, a compact or loose powder, a solid, or a film.

Embodiment 41. The composition according to any one of embodiments 39-40, wherein said composition is toothpastes, toothgels, capsules, soft-gel capsules, tablets such as oral care tablets, oral care films, mouthwashes, oral sprays, sweets such as candies.

Embodiment 42. The composition according to any one of embodiments 39-41, wherein the content of said polyol in said composition is at least 0,5 % weight, at least 1 % weight, at least 5 % weight, or at least 10 % weight of the total weight of said composition.

Embodiment 43. The composition according to any one of embodiments 39-42, wherein said composition is rinsed off, or is left on, after its administration.

Embodiment 44. The composition according to any one of embodiments 39-43, wherein said composition is applied into, or onto, a dental mould, an oral care patch, a dental floss, or a dental strip.

Embodiment 45. The composition according to any one of embodiments 39-44, wherein said composition comprises less than 1 % weight of dimethylisosorbide of the total weight of said composition or is free of dimethylisosorbide.

Embodiment 46. Use of a polyol for preventing and/or treating diseases and/or symptoms associated with pathogenic microorganisms in a subject in need thereof.

Embodiment 47. The use according to embodiment 46, wherein the pathogenic microorganisms are anaerobic pathogenic microorganisms.

Embodiment 48. The use according to embodiment 47, wherein the anaerobic pathogenic microorganisms are strictly anaerobic pathogenic microorganisms.

Embodiment 49. The use according to any one of embodiments 46-48, wherein the polyol is administered in the oral cavity.

Embodiment 50. The use according to any one of embodiments 46-48, wherein the polyol is administered on the skin, eyes, ears, or genital mucous membranes, of said subject.

Embodiment 51. The use according to any one of embodiments 46-50, wherein said pathogenic microorganisms are bacteria of the genus Fusobacterium.

Embodiment 52. The use according to preceding embodiment 51, wherein the bacteria of the genus Fusobacterium are of the species Fusobacterium nucleatum.

Embodiment 53. The use according to preceding embodiment 51, wherein the bacteria of the genus Fusobacterium are of the species Fusobacterium necrophorum.

Embodiment 54. The use according to preceding embodiment 51, wherein the bacteria of the genus Fusobacterium are of the species Fusobacterium polymorphum.

Embodiment 55. The use according to any one of embodiments 46-50, wherein said pathogenic microorganisms are bacteria of the genus Porphyromonas.

Embodiment 56. The use according to preceding embodiment 55, wherein the bacteria of the genus Porphyromonas are of the species Porphyromonas gingivalis.

Embodiment 57. The use according to any one of embodiments 46-50, wherein said pathogenic microorganisms are bacteria of the genus Veillonella.

Embodiment 58. The use according to preceding embodiment 57, wherein the bacteria of the genus Veillonella are of the species Veillonella parvula.

Embodiment 59. The use according to preceding embodiment 57, wherein the bacteria of the genus Veillonella are of the species Veillonella dispar.

Embodiment 60. The use according to preceding embodiment 57, wherein the bacteria of the genus Veillonella are of the species Veillonella atypica.

Embodiment 61. The use according to any one of embodiments 46-50, wherein said pathogenic microorganisms are bacteria of the genus Parvimonas.

Embodiment 62. The use according to preceding embodiment 61, wherein the bacteria of the genus Parvimonas are of the species Parvimonas micra.

Embodiment 63. The use according to any one of embodiments 46-62, wherein the polyol is an hexitol.

Embodiment 64. The use according to any one of embodiments 46-63, wherein the polyol is a monoanhydrohexitol.

Embodiment 65. The use according to any of the embodiments 46-64, wherein the polyol is selected from 1,4-Sorbitan, 2,5-Mannitan, 2,5-Iditan, 3,6-Sorbitan, or combinations thereof.

Embodiment 66. The use according to any one of embodiments 46-63, wherein the polyol is a dianhydrohexitol.

Embodiment 67. The use according to any one of embodiments 46-63 and 66, wherein the polyol is a 1,4-3,6-dianhydrohexitol.

Embodiment 68. The use according to any one of embodiments 46-63 and 66-67, wherein the polyol is selected from isosorbide, isomannide, or isoidide.

Embodiment 69. The use according to any one of the embodiments 46-63 and 66-68, wherein the polyol is isosorbide.

Embodiment 70. The use according to any one of embodiments 46-69, wherein said diseases and/or symptoms are cancer, brain inflammation, Alzheimer's disease, preterm birth, autoimmune diseases, cardiovascular diseases, diabetes, obesity, insulin resistance, rheumatoid arthritis, inflammatory bowel diseases, or respiratory tract inflammation.

Embodiment 71. The use according to embodiment 70, where said cancer is a tongue cancer, an esophagus cancer, a stomach cancer, an intestine cancer, a colon cancer, or a systemic cancer.

Embodiment 72. The use according to embodiment 70, wherein said cardiovascular diseases are atherosclerosis and/or coronary disease.

Embodiment 73. The use according to any one of embodiments 46-69, wherein said diseases and/or symptoms are inside the oral cavity.

Embodiment 74. The use according to embodiment 73, wherein said diseases and/or symptoms are oral lichen planus, oral cancer, or periodontal diseases.

Embodiment 75. The use according to embodiment 74, wherein said periodontal disease is periodontitis.

Embodiment 76. The use according to embodiment 73, wherein said symptoms are inflamed gums, bleeding gums, pain in the gum, teeth and/or jaw at rest, pain in the gum, teeth and/or jaw during chewing, loose teeth, loss of teeth, fever, halitosis.

Embodiment 77. The use according to any one of embodiments 46-76, wherein said polyol slows down or inhibits the growth of said pathogenic microorganisms.

Embodiment 78. The use according to any one of embodiments 46-76, wherein said polyol slows down the formation and/or reduces a biofilm formed by said pathogenic microorganisms.

Embodiment 79. The use according to embodiment 78, wherein said biofilm is formed in the oral cavity.

Embodiment 80. The use according to any one of embodiments 46-79, wherein said polyol is applied during pathogens's growth conditions and/or during pathogens's survival conditions.

Embodiment 81. The use according to embodiment 80, wherein the pathogen's growth conditions correspond to the period following ingestion of food, such as after a meal, and the pathogen's survival conditions correspond to the condition where no nutrients are available to the subject.

Embodiment 82. The use according to any one of embodiments 46-81, wherein said polyol is administered at least once a day, at least twice a day, at least three times a day, at least four times a day.

Embodiment 83. The use according to any one of embodiments 46-82, wherein said polyol is administered for at least 30 seconds, at least 1 minute, at least 2 minutes, at least 3 minutes, at least 5 minutes, at least 10 minutes, at least 30 minutes, at least 1 hour, at least 4 hours, at least 6 hours, at least 8 hours, at least 12 hours, at least 16 hours, at least 20 hours, at least 24 hours.

Embodiment 84. A method for preventing and/or treating diseases and/or symptoms associated with pathogenic microorganisms in a subject in need thereof, comprising administering an effective amount of the composition as described in any one of embodiments 39-45.

Embodiment 85. The method according to embodiment 84, wherein the pathogenic microorganisms are anaerobic pathogenic microorganisms.

Embodiment 86. The method according to embodiment 85, wherein the anaerobic pathogenic microorganisms are strictly anaerobic pathogenic microorganisms.

Embodiment 87. The method according to any one of embodiments 84-86, wherein the polyol is administered in the oral cavity.

Embodiment 88. The method according to any one of embodiments 84-86, wherein the polyol is administered on the skin, eyes, ears, or genital mucous membranes, of said subject.

Embodiment 89. The method according to any one of embodiments 84-88, wherein said pathogenic microorganisms are bacteria of the genus Fusobacterium.

Embodiment 90. The method according to preceding embodiment 89, wherein the bacteria of the genus Fusobacterium are of the species Fusobacterium nucleatum.

Embodiment 91. The method according to preceding embodiment 89, wherein the bacteria of the genus Fusobacterium are of the species Fusobacterium necrophorum.

Embodiment 92. The method according to preceding embodiment 89, wherein the bacteria of the genus Fusobacterium are of the species Fusobacterium polymorphum.

Embodiment 93. The method according to any one of embodiments 84-88, wherein said pathogenic microorganisms are bacteria of the genus Porphyromonas.

Embodiment 94. The method according to preceding embodiment 93, wherein the bacteria of the genus Porphyromonas are of the species Porphyromonas gingivalis.

Embodiment 95. The method according to any one of embodiments 84-88, wherein said pathogenic microorganisms are bacteria of the genus Veillonella.

Embodiment 96. The method according to preceding embodiment 95, wherein the bacteria of the genus Veillonella are of the species Veillonella parvula.

Embodiment 97. The method according to preceding embodiment 95, wherein the bacteria of the genus Veillonella are of the species Veillonella dispar.

Embodiment 98. The method according to preceding embodiment 95, wherein the bacteria of the genus Veillonella are of the species Veillonella atypica.

Embodiment 99. The method according to any one of embodiments 84-88, wherein said pathogenic microorganisms are bacteria of the genus Parvimonas.

Embodiment 100. The method according to preceding embodiment 99, wherein the bacteria of the genus Parvimonas are of the species Parvimonas micra.

Embodiment 101. The method according to any one of embodiments 84-100, wherein the polyol is an hexitol.

Embodiment 102. The use according to any one of embodiments 84-101, wherein the polyol is a monoanhydrohexitol.

Embodiment 103. The method according to any of the embodiments 84-102, wherein the polyol is selected from 1,4-Sorbitan, 2,5-Mannitan, 2,5-Iditan, 3,6-Sorbitan, or combinations thereof.

Embodiment 104. The method according to any one of embodiments 84-101, wherein the polyol is a dianhydrohexitol.

Embodiment 105. The method according to any one of embodiments 84-101 and 104, wherein the polyol is a 1,4-3,6-dianhydrohexitol.

Embodiment 106. The method according to any one of embodiments 84-101 and 104-105, wherein the polyol is selected from isosorbide, isomannide, or isoidide.

Embodiment 107. The method according to any one of embodiments 84-101 and 104-106, wherein the polyol is isosorbide.

Embodiment 108. The method according to any one of embodiments 84-107, wherein said diseases and/or symptoms are cancer, brain inflammation, Alzheimer's disease, preterm birth, autoimmune diseases, cardiovascular diseases, diabetes, obesity, insulin resistance, rheumatoid arthritis, inflammatory bowel diseases, or respiratory tract inflammation.

Embodiment 109. The method according to embodiment 108, where said cancer is a tongue cancer, an esophagus cancer, a stomach cancer, an intestine cancer, a colon cancer, or a systemic cancer.

Embodiment 110. The method according to embodiment 108, wherein said cardiovascular diseases are atherosclerosis and/or coronary disease.

Embodiment 111. The method according to any one of embodiments 84-107, wherein said diseases and/or symptoms are inside the oral cavity.

Embodiment 112. The method according to embodiment 111, wherein said diseases and/or symptoms are oral lichen planus, oral cancer, or periodontal diseases.

Embodiment 113. The method according to embodiment 112, wherein said periodontal disease is periodontitis.

Embodiment 114. The method according to embodiment 111, wherein said symptoms are inflamed gums, bleeding gums, pain in the gum, teeth and/or jaw at rest, pain in the gum, teeth and/or jaw during chewing, loose teeth, loss of teeth, fever, halitosis.

Embodiment 115. The method according to any one of embodiments 84-114, wherein said polyol slows down or inhibits the growth of said pathogenic microorganisms.

Embodiment 116. The method according to any one of embodiments 84-114, wherein said polyol slows down the formation and/or reduces a biofilm formed by said pathogenic microorganisms.

Embodiment 117. The method according to embodiment 116, wherein said biofilm is formed in the oral cavity.

Embodiment 118. The method according to any one of embodiments 84-117, wherein said polyol is applied during pathogens's growth conditions and/or during pathogens's survival conditions.

Embodiment 119. The method according to embodiment 118, wherein the pathogen's growth conditions correspond to the period following ingestion of food, such as after a meal, and the pathogen's survival conditions correspond to the condition where no nutrients are available to the subject.

Embodiment 120. The method according to any one of embodiments 84-119, wherein said polyol is administered at least once a day, at least twice a day, at least three times a day, at least four times a day.

Embodiment 121. The method according to any one of embodiments 84-120, wherein said polyol is administered for at least 30 seconds, at least 1 minute, at least 2 minutes, at least 3 minutes, at least 5 minutes, at least 10 minutes, at least 30 minutes, at least 1 hour, at least 4 hours, at least 6 hours, at least 8 hours, at least 12 hours, at least 16 hours, at least 20 hours, at least 24 hours.

### Examples

### Example 1: in-vitro trials with Fusobacterium nucleatum

The growth of populations of bacteria of the strain *Fusobacterium nucleatum* under two kinds of conditions was determined simultaneously in a culture broth without any isosorbide, i.e. "control culture", and in culture broths containing isosorbide concentrations of 2.5 %weight, 5 %weight and 10%weight. Isosorbide used was Beauté by Roquette^{®} PO 500, sold by the applicant, which is a liquid pure isosorbide with 99%+ of isosorbide.

An experiment consists in an inoculation of a culture broth with a preculture of the bacteria to test, which was prepared 24h or 48h before inoculation. For each experiment, the culture broth was boiled 20 min at 100°C just before inoculation in order to eliminate oxygen.

The "growth conditions" consisted in a culture in a rich brain heart culture medium enriched with 2 g/L cystein chlorhydrate and 10% horse blood for black pigmented anaerobes. The "survival conditions" consisted in a non-nutritive RC broth, i.e. cooked meat medium, enriched with 2 g/L cysteine.

For each experiment, a final volume of culture broth of 10 mL was placed in a tube. Each tube was covered with a paraffin plug to preserve the anaerobic conditions and tubes were placed in an anaerobic jar. Incubation times were 0, 6, 24 and 48 hours. At each incubation time, 0.1 mL of the cultivated medium was sampled and diluted with 0.9 mL RC broth (dilution 10-1) and cascade dilutions were realized until dilution 10-5. A volume of 0.1 mL of each dilution was plated over a cysteinated Columbia gel (plus 10% horse blood for black pigmented anaerobes) and incubated in an anaerobic chamber during 5 days. Then a plate count enumeration was performed. The number of viable bacteria was counted and expressed as LogCFU.mL-1.

Results in growth conditions are presented in the following table 1, and on figure 2 in appendices:

**[Table 1]**

| Incubation time | Count of bacteria (LogCFU/mL) for | | | |
|---|---|---|---|---|
| | Control | 10 %weight Isosorbide | 5 %weight Isosorbide | 2.5 %weight Isosorbide |
| 0 hour | 5.00E+02 | 5.00E+02 | 5.00E+02 | 5.00E+02 |
| 6 hours | 1.00E+03 | 6.00E+03 | 4.10E+04 | 9.00E+03 |
| 24 hours | 5.60E+04 | 1.00E+02 | 1.00E+02 | 1.00E+02 |
| 48 hours | 2.80E+06 | 1.00E+01 | 1.00E+01 | 5.30E+04 |

It was observed that isosorbide first stimulated the growth of *Fusobacterium nucleatum* at 6 hours, and then acted as a biocide at 24 hours for all three isosorbide concentrations, and finally still acted as a biocide at 48 hours.

Results in survival conditions are presented in the following table 2, and on figure 3 in appendices:

**[Table 2]**

| Incubation time | Count of bacteria (LogCFU/mL) for | | | |
|---|---|---|---|---|
| | Control | 10 %weight Isosorbide | 5 %weight Isosorbide | 2.5 %weight Isosorbide |
| 0 hour | 2.50E+05 | 2.50E+05 | 2.50E+05 | 2.50E+05 |
| 6 hours | 4.00E+02 | 1.00E+02 | 1.00E+02 | 1.00E+02 |
| 24 hours | 1.00E+02 | 1.00E+02 | 1.00E+02 | 1.00E+02 |
| 48 hours | 1.00E+02 | 1.00E+02 | 1.00E+02 | 1.00E+02 |

It was observed that isosorbide is not metabolized by *Fusonucleatum bacterium.* It is not a carbon source for this strain. Compared to the control, isosorbide slightly accelerated the decay of *Fusobacterium nucleatum.*

### Example 2: in-vitro trials with Porphyromonas sp.

The growth of populations of bacteria of the strain *Porphyromonas sp.* under two kinds of conditions was determined following the same protocol as for *Fusobacterium nucleatum* in example 1.

Results in growth conditions are presented in the following table 3, and on figure 4 in appendices:

**[Table 3]**

| Incubation time | Count of bacteria (LogCFU/mL) for | | | |
|---|---|---|---|---|
| | Control | 10 %weight Isosorbide | 5 %weight Isosorbide | 2.5 %weight Isosorbide |
| 0 hour | 3.80E+04 | 3.80E+04 | 3.80E+04 | 3.80E+04 |
| 6 hours | 1.57E+06 | 1.00E+03 | 1.00E+03 | 6.40E+04 |
| 24 hours | 2.37E+08 | 1.00E+03 | 7.10E+05 | 4.40E+06 |
| 48 hours | 2.50E+05 | 1.00E+03 | 9.00E+06 | 8.70E+07 |

It was observed that concentrations of isosorbide of 2.5 and 5 %weight first slowed down the growth of *Porphyromonas sp.* at 6 and 24 hours compared to the control, but then stimulated the growth compared to control at 48 hours. A concentration of 10%weight in isosorbide acted as a biocide as soon as 6 hours and up to 48 hours.

Results in survival conditions are presented in the following table 4, and on figure 5 in appendices:

**[Table 4]**

| Incubation time | Count of bacteria (LogCFU/mL) for | | | |
|---|---|---|---|---|
| | Control | 10 %weight Isosorbide | 5 %weight Isosorbide | 2.5 %weight Isosorbide |
| 0 hour | 2.68E+07 | 2.68E+07 | 2.68E+07 | 2.68E+07 |
| 6 hours | 9.10E+03 | 1.00E+02 | 1.00E+02 | 1.00E+02 |
| 24 hours | 1.00E+02 | 1.00E+02 | 1.00E+02 | 1.00E+02 |
| 48 hours | 1.00E+01 | 1.00E+01 | 1.00E+01 | 1.00E+01 |

It was observed that isosorbide is not metabolized by *Porphyromonas sp.* It is not a carbon source for this strain. Compared to the control, isosorbide slightly accelerated the decay of *Porphyromonas sp.*

### Example 3: in-vitro trials with Porphyromonas gingivalis W83.

The growth of populations of bacteria of the strain W83 of *Porphyromonas gingivalis* under two kinds of conditions was determined following the same protocol as for *Fusobacterium nucleatum* in example 1.

Results in growth conditions are presented in the following table 5, and on figure 6 in appendices:

**[Table 5]**

| Incubation time | Count of bacteria (LogCFU/mL) for | | | |
|---|---|---|---|---|
| | Control | 10 %weight Isosorbide | 5 %weight Isosorbide | 2.5 %weight Isosorbide |
| 0 hour | 2.40E+08 | 2.40E+08 | 2.40E+08 | 2.40E+08 |
| 6 hours | 1.60E+06 | 1.00E+02 | 1.00E+02 | 1.00E+02 |
| 24 hours | 1.70E+05 | 1.90E+03 | 6.10E+05 | 1.21E+06 |
| 48 hours | 4.30E+05 | 1.00E+02 | 1.00E+02 | 1.50E+05 |

It was observed that isosorbide acted as a biocide at 2.5, 5 and 10 %weight.

Results in survival conditions are presented in the following table 6, and on figure 7 in appendices:

**[Table 6]**

| Incubation time | Count of bacteria (LogCFU/mL) for | | | |
|---|---|---|---|---|
| | Control | 10 %weight Isosorbide | 5 %weight Isosorbide | 2.5 %weight Isosorbide |
| 0 hour | 6.70E+04 | 6.70E+04 | 6.70E+04 | 6.70E+04 |
| 6 hours | 1.14E+04 | 1.00E+02 | 1.00E+02 | 1.00E+02 |
| 24 hours | 2.30E+03 | 1.00E+02 | 1.00E+02 | 1.00E+02 |
| 48 hours | 1.00E+02 | 1.00E+02 | 1.00E+02 | 1.00E+02 |

It was observed that isosorbide is not metabolized by *Porphyromonas gingivalis W83.* It is not a carbon source for this strain. Compared to the control, isosorbide greatly accelerated the decay of *Porphyromonas gingivalis W83.*

### Example 4: in-vitro trials with Porphyromonas gingivalis D141.

The growth of populations of bacteria of the strain D141 of *Porphyromonas gingivalis* under two kinds of conditions was determined following the same protocol as for *Fusobacterium nucleatum* in example 1.

Results in growth conditions are presented in the following table 7, and on figure 8 in appendices:

**[Table 7]**

| Incubation time | Count of bacteria (LogCFU/mL) for | | | |
|---|---|---|---|---|
| | Control | 10 %weight Isosorbide | 5 %weight Isosorbide | 2.5 %weight Isosorbide |
| 0 hour | 8.50E+03 | 8.50E+03 | 8.50E+03 | 8.50E+03 |
| 6 hours | 3.00E+02 | 1.00E+02 | 1.00E+02 | 6.00E+02 |
| 24 hours | 1.00E+02 | 1.00E+02 | 1.00E+02 | 1.00E+02 |
| 48 hours | 1.00E+02 | 1.00E+02 | 1.00E+02 | 1.00E+02 |

It was observed that isosorbide accelerated the decay of *Porphyromonas gingivalis D141* at 5 and 10 %weight.

Results in survival conditions are presented in the following table 8, and on figure 9 in appendices:

**[Table 8]**

| Incubation time | Count of bacteria (LogCFU/mL) for | | | |
|---|---|---|---|---|
| | Control | 10 %weight Isosorbide | 5 %weight Isosorbide | 2.5 %weight Isosorbide |
| 0 hour | 8.50E+04 | 8.50E+04 | 8.50E+04 | 8.50E+04 |
| 6 hours | 1.00E+02 | 1.00E+02 | 1.00E+02 | 1.00E+02 |
| 24 hours | 1.00E+02 | 1.00E+02 | 1.00E+02 | 1.00E+02 |
| 48 hours | 1.00E+02 | 1.00E+02 | 1.00E+02 | 1.00E+02 |

It was observed that isosorbide is not metabolized by *Porphyromonas gingivalis D141.* It is not a carbon source for this strain. Compared to the control, isosorbide did not accelerate the decay of *Porphyromonas gingivalis D141.*

### Example 5: in-vitro trials with Veillonella parvula.

The growth of populations of bacteria of the strain *Veillonella parvula* under two kinds of conditions was determined following the same protocol as for *Fusobacterium nucleatum* in example 1.

Results in growth conditions are presented in the following table 9, and on figure 10 in appendices:

**[Table 9]**

| Incubation time | Count of bacteria (LogCFU/mL) for | | | |
|---|---|---|---|---|
| | Control | 10 %weight Isosorbide | 5 %weight Isosorbide | 2.5 %weight Isosorbide |
| 0 hour | 6.00E+08 | 6.00E+08 | 6.00E+08 | 6.00E+08 |
| 6 hours | 7.80E+05 | 2.10E+04 | 3.40E+04 | 4.90E+05 |
| 24 hours | 2.00E+07 | 2.20E+04 | 2.40E+06 | 6.40E+06 |
| 48 hours | 8.20E+07 | 2.70E+03 | 5.10E+07 | 1.52E+08 |

It was observed that isosorbide acted as a biocide on *Veillonella parvula* at a concentration of 10 %weight.

Results in survival conditions are presented in the following table 10, and on figure 11 in appendices:

**[Table 10]**

| Incubation time | Count of bacteria (LogCFU/mL) for | | | |
|---|---|---|---|---|
| | Control | 10 %weight Isosorbide | 5 %weight Isosorbide | 2.5 %weight Isosorbide |
| 0 hour | 1.75E+08 | 1.75E+08 | 1.75E+08 | 1.75E+08 |
| 6 hours | 6.60E+05 | 1.08E+05 | 1.20E+05 | 2.20E+05 |
| 24 hours | 1.00E+02 | 1.00E+02 | 1.00E+02 | 1.00E+02 |
| 48 hours | 1.00E+02 | 1.00E+02 | 1.00E+02 | 1.00E+02 |

It was observed that isosorbide is not metabolized by *Veillonella parvula.* It is not a carbon source for this strain. Compared to the control, decay of *Veillonella parvula* with isosorbide is almost the same as the decay of control.

### Example 6: in-vitro trials with Parvimonas micra.

The growth of populations of bacteria of the strain *Parvimonas micra* under two kinds of conditions was determined following the same protocol as for *Fusobacterium nucleatum* in example 1.

Results in growth conditions are presented in the following table 11, and on figure 12 in appendices:

**[Table 11]**

| Incubation time | Count of bacteria (LogCFU/mL) for | | | |
|---|---|---|---|---|
| | Control | 10 %weight Isosorbide | 5 %weight Isosorbide | 2.5 %weight Isosorbide |
| 0 hour | 5.60E+05 | 1.49E+05 | 1.49E+05 | 1.49E+05 |
| 6 hours | 2.80E+06 | 1.50E+04 | 2.10E+04 | 5.60E+03 |
| 24 hours | 1.20E+06 | 3.30E+07 | 5.40E+06 | 6.40E+06 |
| 48 hours | 7.30E+06 | 1.00E+02 | 1.90E+04 | 1.33E+05 |

It was observed that isosorbide first disrupted the growth at 6 hours for all 3 concentrations tested, but then allowed the growth to recover at 24 hours, and finally acted as a biocide at concentrations of 2.5, 5 and 10 %weight, and that the higher the concentration in isosorbide was, the higher the biocide effect was.

Results in survival conditions are presented in the following table 12, and on figure 13 in appendices:

**[Table 12]**

| Incubation time | Count of bacteria (LogCFU/mL) for | | | |
|---|---|---|---|---|
| | Control | 10 %weight Isosorbide | 5 %weight Isosorbide | 2.5 %weight Isosorbide |
| 0 hour | 5.60E+05 | 5.60E+05 | 5.60E+05 | 5.60E+05 |
| 6 hours | 1.90E+03 | 4.60E+04 | 4.50E+04 | 6.60E+04 |
| 24 hours | 1.00E+02 | 1.00E+02 | 1.00E+02 | 1.00E+02 |
| 48 hours | 1.00E+02 | 1.00E+02 | 1.00E+02 | 1.00E+02 |

It was observed that isosorbide is not metabolized by *Parvimonas micra.* It is not a carbon source for this strain. Compared to the control, decay of *Veillonella parvula* at 6 hours with isosorbide was slightly lower than the decay of control and reached the same level after 24 hours.

### Example 7: in-vitro trials with Streptococcus oralis

The growth of populations of bacteria of the strain *Streptococcus oralis* under growth conditions was determined following the same protocol as for *Fusobacterium nucleatum* in example 1. Results in growth conditions are presented in the following table 13.

**[Table 13]**

| Incubation time | Count of bacteria (LogCFU/mL) for | | | |
|---|---|---|---|---|
| | Control | 10 %weight Isosorbide | 5 %weight Isosorbide | 2.5 %weight Isosorbide |
| 0 hour | 3.50E+04 | 3.50E+04 | 3.50E+04 | 3.50E+04 |
| 6 hours | 2.61E+08 | 1.70E+07 | 2.84E+08 | 3.23E+08 |
| 24 hours | 9.50E+08 | 1.80E+08 | 1.90E+08 | 1.36E+08 |
| 48 hours | 8.30E+07 | 2.17E+07 | 7.80E+07 | 3.90E+07 |

It was thus observed that isosorbide at concentrations 2.5-10 %weight did not change the growth of this strain.

### Example 8: in-vitro trials with Streptococcus sanguinis

The growth of populations of bacteria of the strain *Streptococcus sanguinis* under growth conditions was determined following the same protocol as for *Fusobacterium nucleatum* in example 1. Results in growth conditions are presented in the following table 14.

**[Table 14]**

| Incubation time | Count of bacteria (LogCFU/mL) for | | | |
|---|---|---|---|---|
| | Control | 10 %weight Isosorbide | 5 %weight Isosorbide | 2.5 %weight Isosorbide |
| 0 hour | 1.00E+04 | 1.00E+04 | 1.00E+04 | 1.00E+04 |
| 6 hours | 2.60E+07 | 2.40E+06 | 8.60E+06 | 2.00E+07 |
| 24 hours | 3.12E+08 | 3.00E+07 | 1.04E+08 | 1.64E+08 |
| 48 hours | 3.50E+07 | 3.00E+07 | 6.70E+07 | 7.70E+07 |

It was thus observed that isosorbide at concentrations 2.5-10 %weight did not change the growth of this strain.

### Example 9: in-vitro trials with Lactobacillus plantarum

The growth of populations of bacteria of the strain *Lactobacillus plantarum* under growth conditions was determined following the same protocol as for *Fusobacterium nucleatum* in example 1. Results in growth conditions are presented in the following table 15.

**[Table 15]**

| Incubation time | Count of bacteria (LogCFU/mL) for | | | |
|---|---|---|---|---|
| | Control | 10 %weight Isosorbide | 5 %weight Isosorbide | 2.5 %weight Isosorbide |
| 0 hour | 8.20E+03 | 8.20E+03 | 8.20E+03 | 8.20E+03 |
| 6 hours | 1.10E+04 | 3.90E+04 | 3.80E+04 | 4.80E+04 |
| 24 hours | 9.10E+05 | 4.30E+05 | 1.10E+06 | 3.20E+06 |
| 48 hours | 3.10E+06 | 1.70E+06 | 5.90E+06 | 5.50E+06 |

. It was thus observed that isosorbide at concentrations 2.5-10 %weight did not change the growth of this strain.

### Example 10: in-vitro trials with Lactobacillus gasseri

The growth of populations of bacteria of the strain *Lactobacillus gasseri* under growth conditions was determined following the same protocol as for *Fusobacterium nucleatum* in example 1. Results in growth conditions are presented in the following table 16.

**[Table 16]**

| Incubation time | Count of bacteria (LogCFU/mL) for | | | |
|---|---|---|---|---|
| | Control | 10 %weight Isosorbide | 5 %weight Isosorbide | 2.5 %weight Isosorbide |
| 0 hour | 2.20E+04 | 2.20E+04 | 2.20E+04 | 2.20E+04 |
| 6 hours | 7.60E+04 | 1.02E+05 | 9.40E+04 | 1.90E+05 |
| 24 hours | 2.80E+06 | 3.40E+06 | 6.30E+06 | 1.11E+06 |
| 48 hours | 1.10E+06 | 1.04E+06 | 3.10E+06 | 9.50E+05 |

It was thus observed that isosorbide at concentrations 2.5-10 %weight did not change the growth of this strain.

### Citation List

### Patent Literature Documents

For any purpose, the following patent document is cited:
- patcit1: FR1761521 "Use of dianhydrohexitol in oral and dental care to reduce the development of bacterial strains".

## Claims

1. Polyol for use in prevention and/or treatment of diseases and/or symptoms associated with pathogenic microorganisms in a subject in need thereof.

2. The polyol for use according to claim 1, wherein the pathogenic microorganisms are anaerobic pathogenic microorganisms.

3. The polyol for use according to claim 2, wherein the anaerobic pathogenic microorganisms are strictly anaerobic pathogenic microorganisms.

4. The polyol for use according to any one of claims 1-3, wherein said pathogenic microorganisms are bacteria of the genus *Fusobacterium,* such as bacteria of the species *Fusobacterium nucleatum, Fusobacterium necrophorum,* or *Fusobacterium polymorphum.*

5. The polyol for use according to any one of claims 1-3, wherein said pathogenic microorganisms are bacteria of the genus *Porphyromonas,* such as bacteria of the species *Porphyromonas gingivalis.*

6. The polyol for use according to any one of claims 1-3, wherein said pathogenic microorganisms are bacteria of the genus *Veillonella,* such as bacteria of the species *Veillonella parvula, Veillonella dispar,* or *Veillonella atypica.*

7. The polyol for use according to any one of claims 1-3, wherein said pathogenic microorganisms are bacteria of the genus *Parvimonas,* such as bacteria of the species *Parvimonas micra.*

8. The polyol for use according to any of the preceding claims, wherein the polyol is an hexitol.

9. The polyol for use according to any of the preceding claims, wherein the polyol is a monoanhydrohexitol, such as 1,4-Sorbitan, 2,5-Mannitan, 2,5-Iditan, 3,6-Sorbitan, or combinations thereof.

10. The polyol for use according to any one of claims 1-8, wherein the polyol is a dianhydrohexitol, such as a 1,4-3,6-dianhydrohexitol, or such as isosorbide, isomannide, isoidide, or combinations thereof.

11. The polyol for use according to any one of the claims 1-8 and 10, wherein the polyol is isosorbide.

12. The polyol for use according to any one of the preceding claims, wherein said diseases and/or symptoms are cancer, such as a tongue cancer, an esophagus cancer, a stomach cancer, an intestine cancer, a colon cancer, or a systemic cancer ; brain inflammation, Alzheimer's disease, preterm birth, autoimmune diseases, cardiovascular diseases, such as atherosclerosis and/or coronary disease ; diabetes, obesity, insulin resistance, rheumatoid arthritis, inflammatory bowel diseases, or respiratory tract inflammation.

13. The polyol for use according to any one of claims 1-12, wherein said diseases and/or symptoms are inside the oral cavity, such as oral lichen planus, oral cancer, or periodontal diseases such as periodontitis.

14. The polyol for use according to claim 13, wherein said symptoms are inflamed gums, bleeding gums, pain in the gum, teeth and/or jaw at rest, pain in the gum, teeth and/or jaw during chewing, loose teeth, loss of teeth, fever, halitosis.

15. Composition comprising a polyol for use in prevention and/or treatment of diseases and/or symptoms associated with pathogenic microorganisms in a subject in need thereof, as described in claims 1-14.

16. The composition according to claim 15, wherein said composition is a liquid, a gel, a compact or loose powder, a solid, or a film.

17. The composition according to any one of claims 15-16, wherein said composition is a toothpaste, a toothgel, a capsule, a soft-gel capsule, a tablet such as oral care tablet, an oral care film, a mouthwash, an oral spray, or a sweet such as a candy.

18. The composition according to any one of claims 15-17, wherein the content of said polyol in said composition is at least 0,5 % weight, at least 1 % weight, at least 5 % weight, or at least 10 % weight of the total weight of said composition.

19. The composition according to any one of claims 15-18, wherein said composition is rinsed off, or is left on, after its administration.

20. The composition according to any one of claims 15-19, wherein said composition is applied into, or onto, a dental mould, an oral care patch, a dental floss, or a dental strip.

21. The composition according to any one of claims 15-20, wherein said composition comprises less than 1 % weight of dimethylisosorbide of the total weight of said composition or is free of dimethylisosorbide.
